# EUROPEAN PATENT APPLICATION

(11) **EP 4 748 938 A1**
(43) Date of publication of application: **27.05.2026**
(21) Application number: 24842344.4
(22) Date of filing: 15.07.2024
(51) Int. Cl.: C12N 15/82, C12N 15/29, A01H 5/00, A01H 6/46

(54) **METHOD FOR CREATING BROAD-SPECTRUM DISEASE-RESISTANT TRANSGENIC MAIZE**

(30) Priority: 18.07.2023 CN 202310879971
(71) Applicant: Win-all Hi-tech Seed Co., Ltd., Hefei, Anhui 230088 (CN); Shanghai ZKW Molecular Breeding Technology Co., Ltd., Shanghai 200233 (CN); Zhang, Conghe, Hefei, Anhui 230088 (CN)
(72) Inventor: WANG, Hui, Hefei, Anhui 230088 (CN); WANG, Yiqin, Hefei, Anhui 230088 (CN); WANG, Lin, Hefei, Anhui 230088 (CN); FANG, Yu, Hefei, Anhui 230088 (CN); CHU, Chengcai, Hefei, Anhui 230088 (CN); ZHANG, Qin, Hefei, Anhui 230088 (CN); YAN, Zhi, Hefei, Anhui 230088 (CN); ZHU, Quangui, Hefei, Anhui 230088 (CN); ZHANG, Yunhu, Hefei, Anhui 230088 (CN); DONG, Siyuan, Hefei, Anhui 230088 (CN); SHEN, Guangle, Hefei, Anhui 230088 (CN); JI, Huaiyuan, Hefei, Anhui 230088 (CN); ZHANG, Conghe, Hefei, Anhui 230088 (CN)
(74) Representative: Winter, Brandl - Partnerschaft mbB
(86) International application number: PCT/CN2024/105422
(87) International publication number: WO 2025/016353

(57) **Abstract**

Provided is a method for creating broad-spectrum disease-resistant transgenic maize. Specifically provided is a method for preparing a transgenic plant having enhanced disease resistance and/or improved production performance. The method comprises the following steps: introducing a multifunctional gene or a multifunctional gene-related biomaterial into a receptor plant to obtain a transgenic plant having higher disease resistance and/or production performance higher than the receptor plant. The multifunctional gene is a DNA molecule as shown in SEQ ID NO: 1.

## Description

### CROSS-REFERENCE TO RELATED APPLICATION

The present application claims the priority to Chinese Patent Application (Application number: 202310879971.7), filed on July 18, 2023, the entire content of which is incorporated herein by reference.

### Technical Field

The present application belongs to the field of plant genetic engineering, and relates to a method for creating broad-spectrum disease-resistant transgenic maize.

### Background Art

Plant diseases have always been a significant issue that cannot be ignored in agricultural production. They not only lead to reduced crop yields but also cause a decline in the quality of agricultural products, resulting in substantial losses for production, and in severe cases, causing food shortages and a series of social problems.

Maize (Zea mays L.) is a major food crop in many regions of the world. During its breeding and production practice, it has been found that ear rot, southern rust, Helminthosporium maydis and Helminthosporium turcicum (especially ear rot) are main diseases during the growth process of maize. These diseases not only cause direct losses in maize yield but also affect the quality of maize, posing a threat to the safety of maize and its feed.

The ear rot of maize is also known as gibberellic disease or ear dry rot disease. The ears and kernels of maize may all be affected by this disease, wherein top or middle parts of the affected ears change color, and pink, blue-green, black-gray, dark brown or yellowish-brown mold layers appear. That is, thallus, conidiophores and conidia of pathogenic bacteria extend to 1/3-1/2 of the female ear, and may extend to the whole female ear in rainy days or at high humidity. The diseased kernels are dull, unfull, brittle, empty inside, and often filled with intertwined mycelia. The bracts of the ears are often penetrated by dense mycelia, which are bonded together and attached to the ears and are not prone to peel off. The southern rust of maize mainly occurs on the leaves of maize, and can also infect the leaf sheaths, stems and bracts. In severe cases, the entire leaf may be covered with rust-brown disease spots, which cause the leaves to turn yellow, and also harm the bracts, ears and male flowers. The Helminthosporium maydis, also known as a spot disease of maize, can occur throughout the entire growth period of maize, mainly harming leaves, and also leaf sheaths, bracts and ears. If disease spots on the leaves are relatively small and there are many disease spots, a layer of gray molds, i.e., conidiophores and conidia of pathogenic bacteria, is densely formed on the surfaces of the disease spots under high temperature and high humidity conditions. The Helminthosporium turcicum of maize mainly harms leaves, and in severe cases, also harms leaf sheaths and bracts. The leaves on the lower part of the plant are first diseased and then expand upward. The disease spots are each shaped as a long spindle, and present gray-brown or yellow-brown, or large irregular necrotic spots connected by several disease spots. In severe cases, the leaves are scorched.

At present, using genetic engineering means to improve the disease resistance of plants has opened up a brand-new way for disease-resistant breeding and has become the main strategy to alleviate bacterial invasion. In recent years, soybeans, wheat, cotton and other transgenic disease-resistant, insect-resistant and herbicide-resistant crops have been widely promoted and applied, and have achieved huge economic and social benefits. However, due to the unpredictability of a transgenic effect, the same or similar genes may have different effects among different species, and genes that exert a disease-resistant effect in certain species may have no disease-resistant effect in another species, and even lead to reduced yield and other negative effects. Therefore, the research and exploration of genes with significant application value or new functions of known genes, and the successful breeding of excellent high-yield maize varieties resistant to a plurality of diseases by using these genes have become urgent issues to be addressed in maize production. However, no technical reports on the successful research and development of this type of transgenic disease-resistant maize have been seen so far.

### Summary of the application

The successful breeding of excellent high-yield maize varieties resistant to a plurality of diseases has become urgent issues to be addressed in maize production. In order to overcome the defects in the prior art, the present application provides a method for creating broad-spectrum disease-resistant transgenic maize, i.e., preparing a transgenic plant having enhanced disease resistance and/or improved production performance by introducing a multifunctional gene or a multifunctional gene-related biomaterial. The present application has great application and promotion values for the breeding of excellent maize varieties.

### Technical Problem

It is to successfully breed high - yielding and disease - resistant (against multiple diseases) excellent maize varieties.

### Technical solution

The present application provides a method for creating broad-spectrum disease-resistant transgenic maize.

The present application provides use of a multifunctional gene or a multifunctional gene-related biomaterial in the preparation of a transgenic plant having enhanced disease resistance and/or improved production performance.

The present application further provides a method for preparing a transgenic plant having enhanced disease resistance and/or improved production performance, including the following steps: introducing a multifunctional gene or a multifunctional gene-related biomaterial into a receptor plant to obtain a transgenic plant having higher disease resistance and/or production performance than the receptor plant.

Any of the above multifunctional gene is (a1), (a2) or (a3) as follows:
(a1) a DNA molecule as shown in SEQ ID No. 1;
(a2) a DNA molecule that hybridizes with (a1) under stringent conditions; and
(a3) a DNA molecule that has 99% or more identity with (a1).

These stringent conditions may be hybridization and film washing at 65°C in a solution of 0.1×SSPE (or 0.1×SSC) and 0.1% SDS.

The term "identity" as used herein refers to a sequence similarity to a natural nucleic acid sequence. Identity may be evaluated with the naked eyes or computer software. Using the computer software, the identity between two or more sequences may be expressed by percentage (%), which may be used to evaluate the identity between related sequences.

The multifunctional gene-related biomaterial is an expression cassette with the multifunctional gene, a recombinant vector with the multifunctional gene, or a recombinant microorganism with the multifunctional gene.

The recombinant vector may specifically be a recombinant vector constructed with a plant expression vector as a starting vector. The plant expression vector includes a dual Agrobacterium vector and a vector that may be used for plant microprojectile bombardment, etc. The plant expression vector may also contain a 3'-end' untranslated region of a foreign gene, i.e., contain a polyadenylic acid signal and any other DNA fragment involved in mRNA processing or gene expression. When the recombinant vector is constructed using the multifunctional gene of the present application, an enhancer, including a translation enhancer or a transcription enhancer, may also be used. In order to facilitate identification and screening of a transgenic plant cell or plant, the used plant expression vector may be processed, such as adding a gene that may be expressed in a plant and encode an enzyme or luminescent compound that can produce the change in color, a marker gene for antibiotics, or a chemical reagent-resistant marker gene, etc. From the consideration of the safety of transgenic plants, transformed plants may be directly screened in adversity without adding any selective marker genes.

As an example, the recombinant vector is specifically as shown in SEQ ID NO: 2.

Any of the above enhanced disease resistance refers to enhanced disease resistance to ear rot and/or southern rust and/or Helminthosporium maydis and/or Helminthosporium turcicum.

Specifically, the ear rot is ear rot of maize.

Specifically, pathogenic bacteria of the ear rot of maize are Fusarium verticillioides.

Specifically, the southern rust is southern rust of maize.

Specifically, pathogenic bacteria of the southern rust are Puccinia polysora underw.

Specifically, the Helminthosporium maydis is Helminthosporium maydis of maize.

Specifically, pathogenic bacteria of the Helminthosporium maydis are Bipolaris maydis.

Specifically, the Helminthosporium turcicum is Helminthosporium turcicum of maize.

Specifically, pathogenic bacteria of the Helminthosporium turcicum are Exserohlium turcicum.

Any of the above plant may be a monocotyledonous plant or a dicotyledonous plant.

Any of the above plant may be a gramineous plant.

Any of the above plant may be a Zea plant.

Any of the above gramineous plant is maize.

Specifically, the maize may be maize PH4CV.

Any of the improved production performance refers to improved yield.

The present application further provides a DNA molecule, which is (a1), (a2) or (a3) as follows:
(a1) a DNA molecule as shown in SEQ ID No. 1;
(a2) a DNA molecule that hybridizes with (a1) under stringent conditions; and
(a3) a DNA molecule that has 99% or more identity with (a1).

These stringent conditions may be hybridization and film washing at 65°C in a solution of 0.1×SSPE (or 0.1×SSC) and 0.1% SDS.

The term "identity" as used herein refers to a sequence similarity to a natural nucleic acid sequence. Identity may be evaluated with the naked eyes or computer software. Using the computer software, the identity between two or more sequences may be expressed by percentage (%), which may be used to evaluate the identity between related sequences.

The transgenic maize is obtained by means of overexpression of the multifunctional gene provided by the present application in maize. Compared with non-transgenic wild-type control maize, the transgenic maize has significantly improved disease resistance to ear rot, southern rust, Helminthosporium maydis and Helminthosporium turcicum, greatly increased yield (the yield increase rate of more than 38%), and no significant difference in agronomic traits such as plant height, ear position and plant type. Compared with maize Chang 7-2 which has excellent comprehensive agronomic traits in the prior art, the transgenic maize has significantly improved disease resistance to ear rot, southern rust, Helminthosporium maydis and Helminthosporium turcicum, and significantly increased yield (the yield increase rate of 21.2%-22.8%). Compared with maize Zheng 58 which has excellent comprehensive agronomic traits in the prior art, the transgenic maize has significantly improved disease resistance to ear rot, southern rust, Helminthosporium maydis and Helminthosporium turcicum, and significantly increased yield (the yield increase rate of 14.7%-16.2%). The above results show that the multifunctional gene can increase the yield of maize while improving the broad-spectrum disease resistance of maize.

### Beneficial effects

The present application has great application and promotion values for the breeding of excellent maize varieties.

### Brief Description of the Drawings

FIG. 1 is a schematic diagram of an element of a recombinant plasmid.
FIG. 2 is an exemplary electropherogram of plants which are positive by PCR identification.
FIG. 3 shows exemplary phenotypes of a transgenic maize plant and a control maize plant, which are inoculated with pathogenic bacteria of ear rot.
FIG. 4 shows exemplary phenotypes of a transgenic maize plant and a control maize plant, which are inoculated with pathogenic bacteria of southern rust.
FIG. 5 shows exemplary phenotypes of a transgenic maize plant and a control maize plant, which are inoculated with pathogenic bacteria of Helminthosporium maydis.
FIG. 6 shows exemplary phenotypes of a transgenic maize plant and a control maize plant, which are inoculated with pathogenic bacteria of Helminthosporium turcicum.

### Detailed Description

The present application is described in further detail below in conjunction with the specific embodiments, and the given examples are only for the purpose of clarifying the present application, but not for the purpose of limiting the scope of the present application. The examples provided below may be used as a guide for further improvement by a person of ordinary skill in the art and do not in any way constitute a limitation of the present application.

The experimental methods in the following examples, unless otherwise specified, are conventional methods and are carried out in accordance with the techniques or conditions described in the literatures in the art or in accordance with the product instructions. The materials, reagents, etc. used in the following examples unless otherwise specified, may be obtained commercially. Unless otherwise specified, in the quantitative test in the following examples, three replicate experiments were set, and the results were averaged. Phytagar refers to vegetable gel. Maize PH4CV refers to a selfing line PH4CV of maize. Maize Change 7-2 refers to a selfing line Chang 7-2 of maize. Maize Zheng 58 refers to a selfing line Zheng 58 of maize.

An MS basal salt mixture has a full name of an MS plant medium (MURASHIGE & SKOOG MEDIUM), produced by Beijing QiWei YiCheng Tech Co.,Ltd, with a catalog number of M0221. 1000×B5 vitamin has a full name of Gamborg Vitamin Solution (1000X), produced by Beijing XMJ Scientific Co.,Ltd, with a catalog number of G219. MS basal medium with vitamins has a full name of Murashige & Skoog Basal Medium with Vitamins, produced by Beijing XMJ Scientific Co.,Ltd, with a catalog number of M519. 1000×ERIKSSON Vitamin has a full name of Eriksson Vitamin Solution (1000X), produced by Beijing XMJ Scientific Co.,Ltd, with a catalog number of E330. Picloram is produced by Sangon Bioengineering (Shanghai) Co., Ltd., with a catalog number of A610733.

### Example 1: preparation of transgenic maize plant

### I. Construction of plant expression vector

A broad-spectrum disease-resistant gene was as shown in SEQ ID NO: 1.

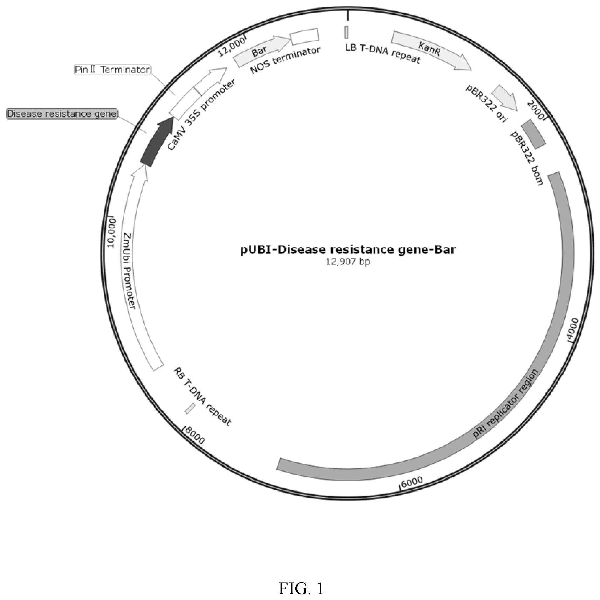

A recombinant plasmid for the expression of the broad-spectrum disease-resistant gene was constructed. The recombinant plasmid was a circular double-stranded DNA molecule, as shown in SEQ ID NO: 2. In SEQ ID NO: 2, positions 425-1219 constituted a kanamycin-resistant gene, positions 10536-11048 constituted a broad-spectrum disease-resistant gene, and positions 11824-12375 constituted a Bar gene. A map of the recombinant plasmid was as shown in FIG. 1.

### II. Genetic transformation of maize

1. The recombinant plasmid constructed in step I was introduced into Agrobacterium EHA105 to obtain recombinant Agrobacterium.
2. The recombinant Agrobacterium obtained in step 1 was propagated, thalli were then collected and resuspended in an inoculation medium containing 200 µM of acetyl syringone, and the OD₆₆₀ₙₘ value of the system was made to 0.5, i.e., an infection solution was obtained.
   The inoculation medium (pH6.0) contained 2.16 g/L MS basal salt mixture, 1 mL/L 1000×B5 vitamin, 68.5 g/L sucrose, 36 g/L glucose, and 0.25 g/L L-proline, the balance being water.
3. Single plants of maize PH4CV were selfed: after about 11 days of pollination, immature ears were collected, surface-sterilized with a 70% ethanol solution for 1 min, and then surface-sterilized with a 1% sodium hypochlorite solution (the concentration was calculated based on the active chlorine content) for 20 min, and immature embryos each having a length of 1.5-2.0 mm were obtained by isolation; and the isolated immature embryos were collected into a 2 mL microcentrifuge tube containing the inoculation medium within 30 min, the inoculation medium was removed with a pipette, 1.5 mL of the infection solution prepared in step 2 was added to the centrifuge tube, and the centrifuge tube was inverted and mixed 5-10 times, and then allowed to stand for 5 min.
4. After step 3 was completed, the immature embryos were taken out, placed on a co-culture medium with its shield facing upward, and cultured at 21°C in the dark for 3 days.
   The co-culture medium (pH5.8) contained 2.16 g/L MS basal salt mixture, 1 mL/L 1000×B5 vitamin, 0.5 mg/L thiamine hydrochloride, 3 mg/L 2,4-D, 10 g/L glucose, 20 g/L sucrose, 0.25 g/L L-proline, 200 µM acetyl syringone, 5 mg/L silver nitrate, and 8 g/L Phytagar, the balance being water.
5. After step 4 was completed, the immature embryos were transferred to a resting medium, and cultured at 28°C in the dark for 7 days.
   The resting medium (pH5.8) contained 4.43 g/L MS basal medium with vitamins, 0.5 mg/L thiamine hydrochloride, 30 g/L sucrose, 0.5 g/L L-proline, 0.5 g/L casamino acid, 1 mg/L 2,4-D, 2.5 mg/L picloram, 5 mg/L silver nitrate, and 3 g/L Phytagar, the balance being water.
6. After step 5 was completed, the immature embryos were transferred to a selective medium, cultured at 28°C in the dark for 21 days, then subcultured to a new selective medium, and cultured at 28°C in the dark for 14 days. During this period, it could be observed that resistant calli developed on the surface of a shield of a zygote.
   The selective medium (pH5.8) contained 4.43 g/L MS basal medium with vitamins, 0.5 mg/L thiamine hydrochloride, 30 g/L sucrose, 0.5 g/L L-proline, 0.5 g/L casamino acid, 1 mg/L 2,4-D, 2.5 mg/L picloram, 5 mg/L silver nitrate, 5 mg/mL bialaphos, and 3 g/L Phytagar, the balance being water.
7. After step 6 was completed, the proliferated resistant calli were transferred to a regenerative medium, and cultured for 14 days. The culture conditions were as follows: the temperature was 26°C, and the photoperiod was 16 h illumination (illumination intensity of 100 µE)/8 h darkness. During this period, green shoots could be observed.
   The regenerative medium (pH5.8) contained 4.33 g/L MS basal salt mixture, 1 mL/L 1000×ERIKSSON vitamin, 3.2 MG/L 6-benzyl adenine, 30 g/L sucrose, 0.5 g/L L-proline, 0.5 g/L casamino acid, 200 mg/mL Timentin, 3 mg/L bialaphos, and 3 g/L Phytagar, the balance being water.
8. After step 7 was completed, healthy shoots were transferred to a culture tube filled with a rooting medium, and cultured for 14 days. The culture conditions were as follows: the temperature was 26°C, and the photoperiod was 16 h illumination (illumination intensity of 100 µE)/8 h darkness.
   The rooting medium (pH5.8) contained 2.22 g/L MS basal medium with vitamins, 10 g/L glucose, 20 g/L maltose, 100 mg/L inositol, 0.2 mg/L indolebutyric acid, 200 mg/mL Timentin, 3 mg/mL bialaphos, 3 g/L phytase, and 3 g/L Phytagar, the balance being water.
9. After step 8 was completed, the rooted plants were transferred to pots filled with peat soil, irrigated with a Hoagland's nutrient solution, then placed in an artificial climate chamber, and cultured for 2 weeks. The culture conditions were as follows: the temperature was 27°C, and the humidity was 70%; and the photoperiod was 16 h illumination (illumination intensity of 3000 lx)/8 h darkness.
10. After step 9 was completed, the plants were transplanted into pots filled with field soil, and placed in an artificial climate chamber for culture, followed by normal cultivation management. The culture conditions were as follows: the temperature was 27°C, and the humidity was 70%; and the photoperiod was 14 h illumination (illumination intensity of 3000 lx)/10 h darkness. These plants were known as T0-generation plants.

### III. Screening and identification of transgenic maize

### 1. Primary screening of transgenic maize

(1) When the T0-generation plants in step II each grew 3-4 true leaves, a first round of screening was carried out.
   Screening method: 1.0 g/100 mL glufosinate-ammonium aqueous solution was smeared to leaf surfaces of seedlings (1 true leaf for each seedling), followed by observation after 5 days. It could be found that the smeared leaves of some single plants of maize turned yellow, the single plants with yellowed leaves were dominant single plants, and all dominant single plants were eliminated.
(2) After the observation of the first round of screening was completed, a second round of screening was carried out.
   Screening method: 1.0 g/100 mL glufosinate-ammonium aqueous solution was smeared to leaf surfaces of seedlings (1 true leaf for each seedling, and this true leaf was not the true leaf used for smearing in the first round of screening), followed by observation after 5 days. It could be found that the smeared leaves of some single plants of maize turned yellow, the single plants with yellowed leaves were dominant single plants, and all dominant single plants were eliminated.
(3) After the observation of the second round of screening was completed, a third round of screening was carried out.
   Screening method: 1.0 g/100 mL glufosinate-ammonium aqueous solution was smeared to leaf surfaces of seedlings (1 true leaf for each seedling, and this true leaf was not the true leaf used for smearing in the first round of screening and the second round of screening), followed by observation after 5 days. It could be found that the smeared leaves of some single plants of maize turned yellow, the single plants with yellowed leaves were dominant single plants, and all dominant single plants were eliminated.

### 2. Molecular identification of transgenic maize

Test plants: all the retaining uneliminated singles plants after the three rounds of screening in step 1 were completed.

Maize PH4CV plants were used as wild-type control plants for test plants.
(1) Fresh young leaves of the test plants (or control plants) were picked, and a total DNA was extracted by a CTAB method.
(2) PCR amplification was carried out by taking the total DNA obtained in step (1) as a template and using a primer pair composed of G4-F and G4-R. A target sequence was 602 bp. If a 602 bp product was present in the amplification products, it was positive by PCR amplification.
   G4-F: 5'-GCTTCCCCTGCTTCCTCTG-3';
   G4-R: 5'-GCCCACATTATAGTGATTAGCATG-3'.
(3) The PCR amplification product in step (2) was subjected to 1.0% agarose gel electrophoresis.

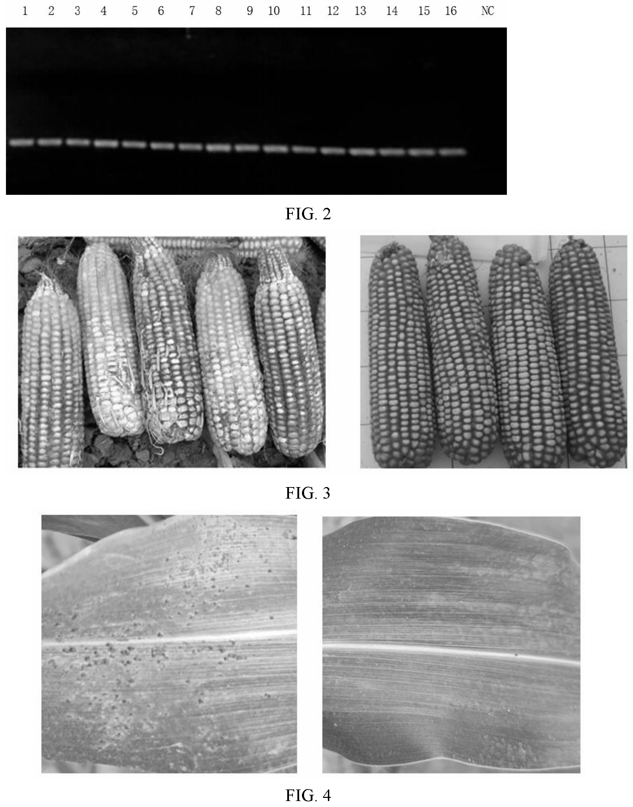

An exemplary electropherogram of plants which were positive by PCR identification was shown in FIG. 2. In FIG. 2, Lanes 1-16 corresponded to different plants which were positive by PCR identification, and NC corresponded to a wild-type control plant.

The plants that were positive by PCR identification were T0-generation transgenic maize plants. Three single plants (i.e., a PH4CV-3 single plant, a PH4CV-11 single plant, and a PH4CV-25 single plant) were randomly selected from the T0-generation transgenic maize plants for Example 2.

Example 2: identification of disease resistance and yield performance of transgenic maize plants Culture conditions in a seedling stage (i.e., before 6-leaf stage) were as follows: the temperature was 26°C, and the humidity was 60%; and the photoperiod was 16 h illumination (illumination intensity of 200 µm/m²/s)/8h darkness.

The culture conditions after the 6-leaf stage were as follows: the temperature was 26°C, and the humidity was 60%; and the photoperiod was 14 h illumination (illumination intensity of 400 µm/m²/s)/10 h darkness.

### I. Acquisition of T1-generation transgenic plants

Single PH4CV-3 plants were selfed to obtain T1-generation seeds. The T1-generation seeds were planted in an artificial climate chamber, followed by normal cultivation management. Molecular identification was carried out at the seedling stage (a method for molecular identification was the same as 2 in step III of Example 1). The plants which were positive by PCR identification were PH4CV-3 T1-generation transgenic plants.

Single PH4CV-11 plants were selfed to obtain T1-generation seeds. The T1-generation seeds were planted in an artificial climate chamber, followed by normal cultivation management. Molecular identification was carried out at the seedling stage (a method for molecular identification was the same as 2 in step III of Example 1). The plants which were positive by PCR identification were PH4CV-11 T1-generation transgenic plants.

Single PH4CV-25 plants were selfed to obtain T1-generation seeds. The T1-generation seeds were planted in an artificial climate chamber, followed by normal cultivation management. Molecular identification was carried out at the seedling stage (a method for molecular identification was the same as 2 in step III of Example 1). The plants which were positive by PCR identification were PH4CV-25 T1-generation transgenic plants.

### II. Acquisition of test plants for disease-resistant test

The disease-resistant test refers to steps III, IV, V and VI.

Test plants: PH4CV-3 T1-generation transgenic plants, PH4CV-11 T1-generation transgenic plants, and PH4CV-25 T1-generation transgenic plants, which were cultured in parallel in the artificial climate chamber. The maize PH4CV plants cultured at the same period and under the same conditions were set as wild-type control plants. Maize Chang 7-2 plants and maize Zheng 58 plants cultured at the same period and under the same conditions were set as existing germplasm control plants.

### III. Disease-resistant test for ear rot

Pathogenic bacteria of the ear rot of maize were Fusarium verticillioides.

When the test plants were cultured to a milky maturity stage, the pathogenic bacteria were inoculated using a conidium suspension inoculation method. The specific steps were as follows: the bracts of maize were surface-sterilized, and an awl was horizontally pierced into the axis of the ear from the inclined top in the middle of the female ear. After the awl was pulled out, 2 mL of spore suspension (a spore suspension containing 1×10⁶ conidia/mL of pathogenic bacteria of ear rot of maize and Tween-80 with a volume percentage of 0.05%, the balance being sterile water) was injected into the hole, and the hole was sealed with cotton soaked in sterile water. One female ear was inoculated for each test plant.

30 days after the completion of inoculation, the maize ears grown from the inoculated female ears were taken as survey objects, and the resistance evaluation was carried out according to diseased areas on the maize ears. The resistance evaluation criteria were shown in Table 1. The resistance evaluation results were shown in Table 2 (10 plants for each maize germplasm were counted). Exemplary photographs of susceptible maize ears and resistant maize ears were shown in FIG. 3 (the left plot showed ears on wild-type control plants, and the right plot showed ears on PH4CV-3 T1-generation transgenic plants).

**Table 1 Resistance evaluation criteria of ear rot of maize**

| Grade of condition | Evaluation criteria | Resistance criteria |
|---|---|---|
| 1 | The diseased area accounted for 0%-1% of the ear area | High resistance (HR) |
| 3 | The diseased area accounted for 2%-10% of the ear area | Resistance (R) |
| 5 | The diseased area accounted for 11%-25% of the ear area | Moderate resistance (MR) |
| 7 | The diseased area accounted for 26%-50% of the ear area | Susceptible (S) |
| 9 | The diseased area accounted for 51%-100% of the ear area | High susceptible (HS) |

**Table 2 Resistance evaluation criteria of ear rot of maize**

| | Phenotype | Resistance evaluation results |
|---|---|---|
| PH4CV-3 T1-generation transgenic plants | The diseased area accounted for 0.5% of the ear area | High resistance (HR) |
| PH4CV-11 T1-generation transgenic plants | The diseased area accounted for 0.24% of the ear area | High resistance (HR) |
| PH4CV-25 T1-generation transgenic plants | The diseased area accounted for 0.7% of the ear area | High resistance (HR) |
| Maize PH4CV plants | The diseased area accounted for 48% of the ear area | Susceptible (S) |
| Maize Change 7-2 plants | The diseased area accounted for 13% of the ear area | Moderate resistance (MR) |
| Maize Zheng 58 plants | The diseased area accounted for 21 % of the ear area | Moderate resistance (MR) |

The identification results showed that the transgenic maize had higher resistance to ear rot than the wild-type control and the existing maize germplasm control, indicating that the broad-spectrum disease-resistant gene as shown in SEQ ID NO: 1 could significantly improve the disease resistance of maize to ear rot.

### IV: Disease-resistant test for southern rust

Pathogenic bacteria of the southern rust were Puccinia polysora underw.

When the test plants were cultured to a pollen release stage, they were subjected to spray inoculation with a spore suspension (a spore suspension containing 1×10⁵ spores of the pathogenic bacteria of southern rust and Tween-80 with a volume percentage of 0.05%, the balance being sterile water). One female ear was sprayed for each plant (the volume of the spore suspension used for spraying was 2 mL).

After 20 days of inoculation, the three leaves above the inoculated female ear and the three leaves below the inoculated female ear were surveyed for resistance evaluation. The resistance evaluation criteria were shown in Table 3. The resistance evaluation results were shown in Table 4 (10 plants for each maize germplasm were counted). Exemplary photographs of susceptible maize leaves and resistant maize leaves were shown in FIG. 4 (the left plot showed leaves on wild-type control plants, and the right plot showed leaves on PH4CV-3 T1-generation transgenic plants).

**Table 3 Resistance evaluation criteria of southern rust of maize**

| Grade of condition | Evaluation criteria | Resistance criteria |
|---|---|---|
| 1 | There were no or very few sori on the leaves | High resistance (HR) |
| 3 | There were a few sori on the leaves, accounting for less than 25% of the leaf area | Resistance (R) |
| 5 | Sori on the leaves accounted for 26%-50% of the leaf area | Moderate resistance (MR) |
| 7 | There were a large number of sori on the leaves, accounting for 51%-75% of the leaf area | Susceptible (S) |
| 9 | There were a large number of sori on the leaves, accounting for 76%-100% of the leaf area | High susceptible (HS) |

**Table 4 Resistance identification results of southern rust of maize**

| | Phenotype | Resistance evaluation results |
|---|---|---|
| PH4CV-3 T1-generation transgenic plants | There were no sori on the leaves | High resistance (HR) |
| PHA4CV-11 T1-generation transgenic plants | There were very few sori on the leaves | High resistance (HR) |
| PH4CV-25 T1-generation transgenic plants | There were no sori on the leaves | High resistance (HR) |
| Maize PH4CV plants | There were a large number of sori on the leaves, accounting for 59% of the leaf area | Susceptible (S) |
| Maize Change 7-2 plants | There were a large number of sori on the leaves, accounting for 62% of the leaf area | Susceptible (S) |
| Maize Zheng 58 plants | There were a large number of sori on the leaves, accounting for 55% of the leaf area | Susceptible (S) |

The identification results showed that the transgenic maize had higher resistance to southern rust than the wild-type control and the existing maize germplasm control, indicating that the broad-spectrum disease-resistant gene as shown in SEQ ID NO: 1 could significantly improve the resistance of maize to southern rust.

### V. Disease-resistant test for Helminthosporium maydis

Pathogenic bacteria of Helminthosporium maydis were Bipolaris maydis.

When the test plants were cultured to a big bell mouth stage, they were subjected to spray inoculation with a spore suspension (a spore suspension containing 1×10⁵ conidia/mL of the pathogenic bacteria of Helminthosporium maydis and Tween-80 with a volume percentage of 0.05%, the balance being sterile water). One female ear was sprayed for each plant (the volume of the spore suspension used for spraying was 2 mL).

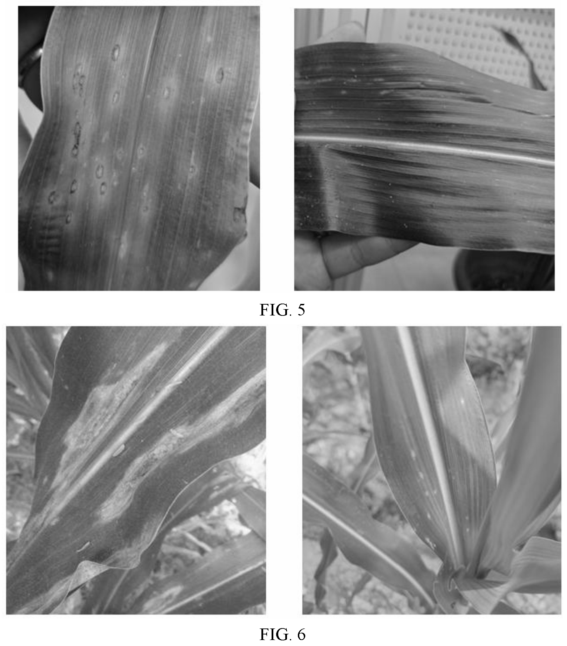

After 10 days of inoculation, the three leaves above the inoculated female ear and the three leaves below the inoculated female ear were surveyed for resistance evaluation. The resistance evaluation criteria were shown in Table 5. The resistance evaluation results were shown in Table 6 (10 plants for each maize germplasm were counted). Exemplary photographs of susceptible maize leaves and resistant maize leaves were shown in FIG. 5 (the left plot showed leaves on wild-type control plants, and the right plot showed leaves on PH4CV-3 T1-generation transgenic plants).

**Table 5 Resistance evaluation criteria of Helminthosporium maydis of maize**

| Grade of condition | Evaluation criteria | Resistance criteria |
|---|---|---|
| 1 | Disease resistance, no disease spots on the leaves or only sporadic disease spots on the lower leaves of the ear position, the disease spots accounting for ≤5% of the leaf area | High resistance (HR) |
| 3 | There were a small number of disease spots on the lower leaves of the ear position, accounting for 6%-10% of the leaf area, and there were sporadic disease spots on the upper leaves of the ear position | Resistance (R) |
| 5 | There were many disease spots on the lower leaves of the ear position, accounting for 11 %-30% of the leaf area, and there were a small number of disease spots on the upper leaves of the ear position | Moderate resistance (MR) |
| 7 | There were a large number of disease spots on the lower leaves of the ear position or the upper leaves of the ear position, the disease spots being connected with each other and accounting for 31%-70% of the leaf area | Susceptible (S) |
| 9 | The leaves of the whole plant were basically covered with disease spots, and the leaves died | High susceptible (HS) |

**Table 6 Resistance identification results of Helminthosporium maydis of maize**

| | Phenotype | Resistance evaluation results |
|---|---|---|
| PH4CV-3 T1-generation transgenic plants | There were only sporadic disease spots on the lower leaves of the ear position, the disease spots accounting for 3% of the leaf area | High resistance (HR) |
| PH4CV-11 T1-generation transgenic plants | There were only sporadic disease spots on the lower leaves of the ear position, the disease spots accounting for 4% of the leaf area | High resistance (HR) |
| PH4CV-25 T1-generation transgenic plants | There were only sporadic disease spots on the lower leaves of the ear position, the disease spots accounting for 4% of the leaf area | High resistance (HR) |
| Maize PH4CV plants | There were a large number of disease spots on the lower leaves of the ear position, the disease spots being connected with each other and accounting for 52% of the leaf area | Susceptible (S) |
| Maize Change 7-2 plants | There were a small number of disease spots on the lower leaves of the ear position, accounting for 7% of the leaf area, and there were sporadic disease spots on the upper leaves of the ear position | Resistance (R) |
| Maize Zheng 58 plants | There were a small number of disease spots on the lower leaves of the ear position, accounting for 9% of the leaf area, and there were sporadic disease spots on the upper leaves of the ear position | Resistance (R) |

The identification results showed that the transgenic maize had higher resistance to Helminthosporium maydis than the wild-type control and the existing maize germplasm control, indicating that the broad-spectrum disease-resistant gene as shown in SEQ ID NO: 1 could significantly improve the resistance of maize to Helminthosporium maydis.

### VI. Disease-resistant test for Helminthosporium turcicum

Pathogenic bacteria of Helminthosporium turcicum were Exserohlium turcicum.

When the test plants were cultured to a big bell mouth stage, they were subjected to spray inoculation with a spore suspension (a spore suspension containing 1×10⁵conidia/mL of the pathogenic bacteria of Helminthosporium turcicum and Tween-80 with a volume percentage of 0.05%, the balance being sterile water). One female ear was sprayed for each plant (the volume of the spore suspension used for spraying was 2 mL).

After 14 days of inoculation, the three leaves above the inoculated female ear and the three leaves below the inoculated female ear were surveyed for resistance evaluation. The resistance evaluation criteria were shown in Table 7. The resistance evaluation results were shown in Table 8 (10 plants for each maize germplasm were counted). Exemplary photographs of susceptible maize leaves and resistant maize leaves were shown in FIG. 6 (the left plot showed leaves on wild-type control plants, and the right plot showed leaves on PH4CV-3 T1-generation transgenic plants).

**Table 7 Resistance evaluation criteria of Helminthosporium turcicum of maize**

| Grade of condition | Evaluation criteria | Resistance criteria |
|---|---|---|
| 1 | There were no disease spots on the leaves or only sporadic disease spots on the lower leaves of the ear position, the disease spots accounting for ≤5% of the leaf area | High resistance (HR) |
| 3 | There were a small number of disease spots on the lower leaves of the ear position, accounting for 5%-10% of the leaf area, and there were sporadic disease spots on the upper leaves of the ear position | Resistance (R) |
| 5 | There were many disease spots on the lower leaves of the ear position, accounting for 10%-30% of the leaf area, and there were a small number of disease spots on the upper leaves of the ear position | Moderate resistance (MR) |
| 7 | There were a large number of disease spots on the lower leaves of the ear position or the upper leaves of the ear position, the disease spots being connected with each other and accounting for 30%-70% of the leaf area | Susceptible (S) |
| 9 | The leaves of the whole plant were basically covered with disease spots, and the leaves died | High susceptible (HS) |

**Table 8 Resistance identification results of Helminthosporium turcicum of maize**

| | Phenotype | Resistance evaluation results |
|---|---|---|
| PH4CV-3 T1-generation transgenic plants | Disease spots on the lower leaves of the ear position accounted for 4% of the leaf area, and there were a small number of disease spots on the upper leaves of the ear position | High resistance (HR) |
| PH4CV-11 T1-generation transgenic plants | There were only sporadic disease spots on the lower leaves of the ear position, the disease spots accounting for 4% of the leaf area | High resistance (HR) |
| PH4CV-25 T1-generation transgenic plants | There were only sporadic disease spots on the lower leaves of the ear position, the disease spots accounting for 5% of the leaf area | High resistance (HR) |
| Maize PH4CV plants | There were a large number of disease spots on the lower leaves of the ear position or the upper leaves of the ear position, the disease spots being connected with each other and accounting for 45% of the leaf area | Susceptible (S) |
| Maize Change 7-2 plants | Disease spots on the lower leaves of the ear position accounted for 7% of the leaf area, and there were a small number of disease spots on the upper leaves of the ear position | Resistance (R) |
| Maize Zheng 58 plants | There were many disease spots on the lower leaves of the ear position, accounting for 9% of the leaf area, and there were a small number of disease spots on the upper leaves of the ear position | Resistance (R) |

The identification results showed that the transgenic maize had higher resistance to Helminthosporium turcicum than the wild-type control and the existing maize germplasm control, indicating that the broad-spectrum disease-resistant gene as shown in SEQ ID NO: 1 could significantly improve the resistance of maize to Helminthosporium turcicum.

### VII. Yield test

The transgenic maize (PH4CV-3 T1-generation transgenic plants, PH4CV-11 T1-generation transgenic plants, and PH4CV-25 T1-generation transgenic plants) which was subjected to molecular identification, and the control plants in the same stage were transplanted to a test field of Nangang Base of Quanyin Academy of Agricultural Sciences of Anhui Winall Hi-tech Seed Co.,Ltd. Control plants mean that maize Change PH4CV plants were used as mild-type control plants, and maize Chang 7-2 plants and maize Zheng 58 plants were used as existing germplasm control plants. The planting area of each maize germplasm was 20 m², and the planting density was 4500 plants/mu. Normal cultivation management was carried out under parallel conditions. The plant type and ear position (cm) of each plant were observed, and the plant height (cm) and yield per plant (g) of each test plant were measured and counted at the maturity stage. Yield per mu (Kg) = yield per plant×4500÷1000. The yield increase rate of the transgenic maize relative to maize PH4CV, the yield increase rate of the transgenic maize relative to maize Chang 7-2 and the yield increase rate of the transgenic maize relative to maize Zheng 58 were calculated.

The results of agronomic traits and yield observed in field planting were shown in Table 9. Compared with maize PH4CV as wild-type control, the transgenic maize has greatly increased yield (the yield increase rate of more than 38%), and no significant difference in agronomic traits such as plant height, ear position and plant type. Compared with maize Chang 7-2 which has excellent comprehensive agronomic traits in the prior art, the transgenic maize has significantly increased yield (the yield increase rate of 21.2%-22.8%). Compared with maize Zheng 58 which has excellent comprehensive agronomic traits in the prior art, the transgenic maize has significantly increased yield (the yield increase rate of 14.7%-16.2%). The results showed that the broad-spectrum disease-resistant gene as shown in SEQ ID NO: 1 could significantly increase the maize yield.

**Table 9 Survey results of yield and agronomic traits**

| | PH4CV-3 T1-generation transgenic plants | PH4CV-11 T1-generation transgenic plants | PH4CV-25 T1-generation transgenic plants | Maize PH4CV plants | Maize Chang 7-2 plants | Maize Zheng 58 plants |
|---|---|---|---|---|---|---|
| Plant height (cm) | 221 | 218 | 224 | 220 | 180 | 190 |
| Ear position (cm) | 76 | 75 | 76 | 76 | 81 | 68 |
| Plant type | Semi-compact | Semi-compact | Semi-compact | Semi-compact | Compact | Compact |
| Yield per plant (g) | 98.5 | 99.3 | 99.8 | 71.1 | 79.5 | 85.9 |
| Equivalent yield per mu (Kg) | 443.3 | 446.9 | 449.1 | 320.0 | 365.7 | 386.6 |
| Yield increase rate compared to PH4CV plants | 38.5% | 39.7% | 40.3% | 0.00% | 14.3% | 20.8% |
| Yield increase rate compared to Change 7-2 plants | 21.2% | 22.2% | 22.8% | -12.5% | 0.00% | 5.7% |
| Yield increase rate compared to Zheng 58 plants | 14.7% | 15.6% | 16.2% | -17.2% | -5.4% | 0.00% |

The present application is detailed above. For a person skilled in the art, the present application may be implemented within a wide range under the same parameters, concentrations and conditions without departing from the purpose and scope of the present application and without unnecessary experiments. Although special examples are given in the present application, it should be understood that further improvements may be made to the present application. In summary, according to the principles of the present application, the present application is intended to include any change, use or improvement of the present application, including changes that deviate from the scope disclosed in the present application and are made by conventional technologies known in the art. According to the scope of the claims attached below, some basic features can be applied.

### Industrial Applicability

The present application discloses a method for preparing a transgenic plant having enhanced disease resistance and/or improved production performance by introducing a multifunctional gene or a multifunctional gene-related biomaterial, which may be used for germplasm improvement of plants, especially germplasm improvement of maize. The present application has great application and promotion values for the breeding of excellent maize varieties.

## Claims

1. Use of a multifunctional gene or a multifunctional gene-related biomaterial in the preparation of a transgenic plant having enhanced disease resistance and/or improved production performance; the multifunctional gene being (a1), (a2) or (a3) as follows:
(a1) a DNA molecule as shown in SEQ ID No. 1;
(a2) a DNA molecule that hybridizes with (a1) under stringent conditions; and
(a3) a DNA molecule that has 99% or more identity with (a1); and
the multifunctional gene-related biomaterial being an expression cassette with the multifunctional gene, a recombinant vector with the multifunctional gene, or a recombinant microorganism with the multifunctional gene.

2. The use according to claim 1, wherein the plant is a gramineous plant.

3. The use according to claim 2, wherein the gramineous plant is maize.

4. The use according to claims 1-3, wherein the enhanced disease resistance refers to enhanced disease resistance to ear rot and/or southern rust and/or Helminthosporium maydis and/or Helminthosporium turcicum.

5. The use according to claims 1-3, wherein the improved production performance refers to improved yield.

6. A method for preparing a transgenic plant having enhanced disease resistance and/or improved production performance, comprising the following steps: introducing a multifunctional gene or a multifunctional gene-related biomaterial into a receptor plant to obtain a transgenic plant having higher disease resistance and/or production performance than the receptor plant;
the multifunctional gene being (a1), (a2) or (a3) as follows:
(a1) a DNA molecule as shown in SEQ ID No. 1;
(a2) a DNA molecule that hybridizes with (a1) under stringent conditions; and
(a3) a DNA molecule that has 99% or more identity with (a1); and
the multifunctional gene-related biomaterial being an expression cassette with the multifunctional gene, a recombinant vector with the multifunctional gene, or a recombinant microorganism with the multifunctional gene.

7. The method according to claim 6, wherein the plant is a gramineous plant.

8. The method according to claim 6 or 7, wherein the enhanced disease resistance refers to enhanced disease resistance to ear rot and/or southern rust and/or Helminthosporium maydis and/or Helminthosporium turcicum.

9. The method according to claim 6 or 7, wherein the improved production performance refers to improved yield.

10. A DNA, which is (a1), (a2) or (a3) as follows:
(a1) a DNA molecule as shown in SEQ ID No. 1;
(a2) a DNA molecule that hybridizes with (a1) under stringent conditions; and
(a3) a DNA molecule that has 99% or more identity with (a1).
